(19) Europäisches Patentamt
European Patent Office
Office européen des brevets

(11) Veröffentlichungsnummer: **0 097 273**
**B1**

(12) # EUROPÄISCHE PATENTSCHRIFT

(45) Veröffentlichungstag der Patentschrift: **19.02.86**

(51) Int. Cl.[4]: **C 07 C 127/17**, C 07 C 127/19, C 07 C 157/07, C 07 C 157/09, A 01 N 47/32

(21) Anmeldenummer: **83105537.1**

(22) Anmeldetag: **06.06.83**

(54) **Benzylharnstoff-Derivate, Verfahren zu ihrer Herstellung und Fungizide für Landwirtschaft und Gartenbau.**

(30) Priorität: **17.06.82 JP 103004/82**

(43) Veröffentlichungstag der Anmeldung: **04.01.84 Patentblatt 84/1**

(45) Bekanntmachung des Hinweises auf die Patenterteilung: **19.02.86 Patentblatt 86/8**

(84) Benannte Vertragsstaaten: **BE CH DE FR GB IT LI NL**

(56) Entgegenhaltungen:
**EP - A - 0 000 376**
**FR - A - 2 359 123**
**US - A - 3 174 843**
**US - A - 3 304 167**

**Die Akte enthält technische Angaben, die nach dem Eingang der Anmeldung eingereicht wurden und die nicht in dieser Patentschrift enthalten sind.**

(73) Patentinhaber: **NIHON TOKUSHU NOYAKU SEIZO K.K., No.4, 2-Chome, Nihonbashi Honcho, Chuo-ku Tokyo 103 (JP)**

(72) Erfinder: **Yamada, Yasuo, 5-15-9, Nishihirayama, Hino-shi Tokyo (JP)**
Erfinder: **Saito, Junichi, 3-7-12, Osawa, Mitaka-shi Tokyo (JP)**
Erfinder: **Tamura, Tatsuo, 1-7-3, Hanenaka Hamura-machi, Nishitama-gun Tokyo (JP)**
Erfinder: **Sakawa, Shinji, 1-14-13, Tamadaira, Hino-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

**EP 0 097 273 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Benzylharnstoff-Derivate, ein Verfahren zu ihrer Herstellung und Fungizide für Landwirtschaft und Gartenbau.

Insbesondere betrifft die vorliegende Erfindung ein Benzylharnstoff-Derivat der allgemeinen Formel (I)

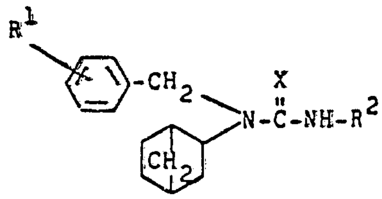
(I)

in der

$R^1$ ein Halogen-Atom oder eine Alkyl-Gruppe mit 1 bis 4 Kohlenstoffatomen bezeichnet,

X ein Sauerstoff- oder Schwefel-Atom bezeichnet und

$R^2$ eine Alkyl-Gruppe mit 1 bis 8 Kohlenstoffatomen, eine Cycloalkyl-Gruppe mit 5 bis 8 Kohlenstoffatomen oder eine Phenyl-Gruppe, die durch mindestens einen Substituenten aus der aus Halogen-Atomen, Alkyl-Gruppen mit 1 bis 4 Kohlenstoffatomen, Alkoxy-Gruppen mit 1 bis 4 Kohlenstoffatomen und Hydroxy-Gruppen bestehenden Klasse substituiert sein kann, bezeichnet.

Die Verbindung der allgemeinen Formel (I) kann mittels der folgenden Verfahren hergestellt werden, auf die sich die vorliegende Erfindung ebenfalls bezieht.

*Verfahren i)*

Verfahren zur Herstellung des Benzylharnstoff-Derivats der allgemeinen Formel (I), bei dem ein Amin der allgemeinen Formel

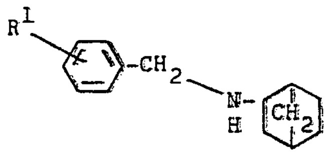
(II)

in der $R^1$ die im Vorstehenden angegebenen Bedeutung hat, mit einem Isocyanat der allgemeinen Formel

$$R^2-N=C=X \qquad (III)$$

in der X und $R^2$ die im Vorstehenden angegebenen Bedeutungen haben, zur Umsetzung gebracht wird.

*Verfahren ii)*

Verfahren zur Herstellung des Benzylharnstoff-Derivats der allgemeinen Formel (I), bei dem ein Carbamoylhalogenid der allgemeinen Formel

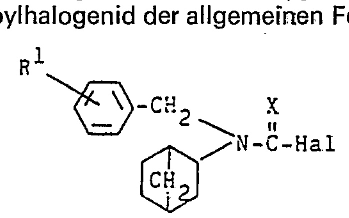
(IV)

in der $R^1$ und X die im Vorstehenden angegebenen Bedeutungen haben, mit einem Amin der allgemeinen Formel

$$H_2N-R^2 \qquad (V)$$

in der $R^2$ die im Vorstehenden angegebene Bedeutung hat, zur Umsetzung gebracht wird.

Die vorliegende Erfindung betrifft weiterhin Fungizide für Landwirtschaft und Gartenbau, die das Benzylharnstoff-Derivat der allgemeinen Formel (I) als Wirkstoff enthalten.

Seitens der Anmelderin wurden ausgedehnte Untersuchungen mit dem Ziel der Gewinnung neuer Verbindungen mit fungizider Wirksamkeit angestellt. Im Zuge dieser Untersuchungen wurde nunmehr gefunden, dass die hierin offenbarten neuen Benzylharnstoff-Derivate der allgemeinen Formel (I), die bisher in der Literatur nicht beschrieben sind, eine hervorragende fungizide Wirksamkeit für Zwecke der Anwendung in Landwirtschaft und Gartenbau besitzen.

Im Rahmen der Arbeiten der Anmelderin wurde gefunden, dass die Verbindungen der Formel (I) eine ausgezeichnete Bekämpfungswirkung gegen Schalenmehltau (Pellicularia sasakii; «sheath blight»), neben der Brusone-Krankheit (Piricularia oryzae Cavara) die wichtigste Reis-Krankheit, zeigen und zur Pilzbekämpfung im Reisanbau durch Sprühbehandlung von Stengeln und Blättern oder Oberflächenbewässerung mit Vorteil eingesetzt werden können.

Weiterhin wurde gefunden, dass die Verbindungen der vorliegenden Erfindung eine herausragende, bisher nicht beobachtete Bekämpfungswirkung in der Weise zeigen, dass sie eine ungewöhnliche Fähigkeit zum Eindringen und zur Wanderung und damit Ausbreitung besitzen, wenn sie auf die Wasseroberfläche gebracht werden. Infolgedessen sind die Verbindungen der vorliegenden Erfindung dadurch von grossem industriellen Wert, dass sie zu einer Einsparung bei dem zur Durchführung der Wasser-Oberflächenbehandlung erforderlichen Arbeitsaufwand führen.

Darüber hinaus wurde gefunden, dass die Verbindungen gemäss der vorliegenden Erfindung eine hervorragende Bekämpfungswirkung auch gegen die von Rhizoctonia solani verursachte Triebfäule bei Gemüsearten zeigen, die in den letzten Jahren ein Problem darstellt.

Die charakteristischen Eigenschaften der Verbindugen gemäss der vorliegenden Erfindung liegen nicht nur in ihrem biologischen Verhalten, sondern auch in ihrer chemischen Struktur. Die Verbindungen der Erfindung sind speziell, wie die allgemeine Formel (I) erkennen lässt, dadurch gekennzeichnet, dass sie als Grundskelett eine Harnstoff- oder Thioharnstoff-Struktur enthalten, und diese Grundstruktur trägt eine substituierte Benzyl-Gruppe an dem Stickstoff-Atom in 1-Stellung, eine Alkylgruppe mit 1 bis 8 Kohlenstoffatomen, eine Cycloalkyl-Gruppe mit 5 bis 8 Kohlenstoffatomen oder eine substituierte Phenyl-Gruppe an dem Stickstoff-Atom in 3-Stellung sowie, und dies ist das besondere individuelle kennzeichnende Merkmal der Verbindungen der vorliegenden

Erfindung, eine Norbornyl-Gruppe an dem Stickstoff-Atom in 1-Stellung.

Es wurde nunmehr gefunden, dass die Verbindungen gemäss der vorliegenden Erfindung, die eine solche ungewöhnliche Struktur aufweisen und nicht in der bisher bekannten Literatur beschrieben sind, leicht synthetisiert werden können und aufgrund ihrer neuartigen chemischen Struktur eine herausragende fungizide Aktivität zeigen.

Ziel dieser Erfindung ist demgemäss, die neuen Benzylharnstoff-Derivate der allgemeinen Formel (I) und Verfahren zu ihrer Herstellung verfügbar zu machen und ihre Verwendung als Fungizide für landwirtschaft und Gartenbau zu ermöglichen.

Weitere Ziele und Vorteile der vorliegenden Erfindung sind der folgenden Beschreibung zu entnehmen.

Die aktiven Verbindungen gemäss der vorliegenden Erfindung können eingesetzt werden gegen pathogene Pilze auf den unterirdischen und bodennahen Pflanzenteilen, pathogene Pilze, die Pflanzen vom Boden her angreifen und Tracheomycose verursachen, sich auf den Sämlingen ausbreitende pathogene Pilze sowie sich im Boden ausbreitende pathogene Pilze.

Da diese Verbindungen sich weiterhin dadurch auszeichnen, dass sie nur eine niedrige Toxizität gegenüber warmblütigen Tieren besitzen und für höhere Pflanzen gut verträglich sind, das heisst, bei den üblichen Dosierungen keine Phytotoxizität gegen Kulturpflanzen zeigen, lassen sie sich sehr vorteilhaft als Chemikalien zur Bekämpfung von Pflanzenkrankheiten, die durch pathogene Pilze hervorgerufen werden, in Landwirtschaft und Gartenbau einsetzen.

Als Fungizide können die Verbindungen der Formel (I) gemäss der vorliegenden Erfindung in wirksamer Weise zur Bekämpfung verschiedenartiger Pilzkrankheiten eingesetzt werden, die durch Archimycetes, Phycomycetes, Ascomycetes, Basidiomycetes und Fungi imperfecti verursacht werden, sowie auch gegen durch Bakterien verursachte Pflanzenkrankheiten.

Die Benzylharnstoff-Derivate der vorliegenden Erfindung, die durch die Formel (I) bezeichnet werden, können mittels der nachstehenden Verfahren i) und ii) hergestellt werden.

*Verfahren i)*

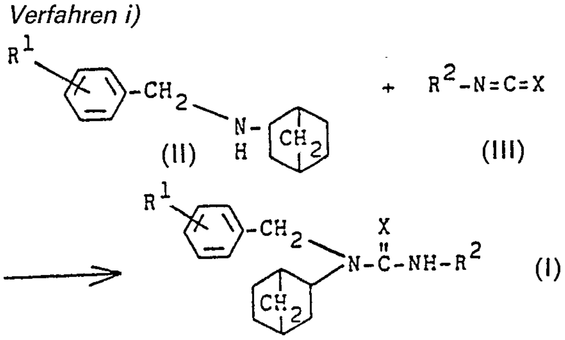

(II) (III) (I)

In den Formeln haben $R^1$, X und $R^2$ die im Vorstehenden angegebenen Bedeutungen.

Spezielle Beispiele für $R^1$ sind Halogen-Atome wie Fluor, Chlor, Brom und Iod, und Alkyl-Gruppen mit 1 bis 4 Kohlenstoff-Atomen wie die Methyl-, Ethyl-, Propyl-, Isopropyl- und n-(iso-, sec- oder tert-)Butyl-Gruppe.

X bezeichnet ein Sauerstoff- oder Schwefel-Atom.

Zu speziellen Beispielen für $R^2$ zählen Alkyl-Gruppen mit 1 bis 4 Kohlenstoff-Atomen, wie sie im vorstehenden beispielhaft genannt wurden, Alkyl-Gruppen mit 5 bis 8 Kohlenstoff-Atomen wie n-(oder iso-)Pentyl, n-(oder iso-)Hexyl, n-Heptyl und n-Octyl, Cycloalkyl-Gruppen mit 5 bis 8 Kohlenstoff-Atomen wie Cyclopentyl, Cyclohexyl, Cycloheptyl und Cyclooctyl, sowie eine Phenyl-Gruppe, die durch wenigstens einen Substituenten ausgewählt aus der aus den gleichen Halogen-Atomen, wie sie im Vorstehenden beispielhaft genannt wurden, den gleichen Alkyl-Gruppen mit 1 bis 4 Kohlenstoff-Atomen, wie sie im Vorstehenden beispielhaft genannt wurden, Alkoxy-Gruppen mit den gleichen Alkyl-Gruppen, wie sie im Vorstehenden beispielhaft genannt wurden, und Hydroxyl-Gruppen bestehenden Klasse substituiert sein kann.

Zu speziellen Beispielen für das Amin der allgemeinen Formel (II) als Ausgangsmaterial für das Verfahren zur Herstellung der Verbindungen der vorliegenden Erfindung, wie sie in dem vorstehenden Reaktionsschema dargestellt ist, zählen

N-4-Chlorobenzyl-2-norbornylamin,
N-4-Bromobenzyl-2-norbornylamin und
N-4-Methylbenzyl-2-norbornylamin.

Zu speziellen Beispielen für das Isocyanat der allgemeinen Formel (III), das in gleicher Weise ein Ausgangsmaterial ist, zählen

Methylisocyanat,
Ethylisocyanat,
Propylisocyanat,
Isopropylisocyanat,
n-Butylisocyanat,
n-Hexylisocyanat,
Cyclopentylisocyanat,
Cyclohexylisocyanat,
Phenylisocyanat,
2-Chlorophenylisocyanat,
4-Chlorophenylisocyanat,
4-Hydroxyphenylisocyanat,
o-Tolylisocyanat,
p-Tolylisocyanat,
3-Methoxyphenylisocyanat,
Methylisothiocyanat,
Ethylisothiocyanat,
n-Butylisothiocyanat,
Phenylisothiocyanat und
Cyclohexylisothiocyanat.

Das vorstehend genannte Verfahren wird an einem typischen Beispiel im einzelnen beschrieben.

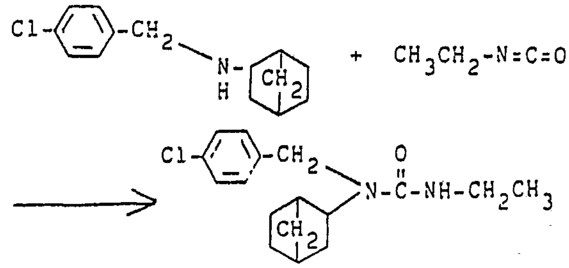

Das vorstehend beschriebene Verfahren kann zweckmässigerweise unter Verwendung eines Lösungsmittels oder Verdünnungsmittels durchgeführt werden. Zu diesem Zweck können sämtliche inerten Lösungsmittel und Verdünnungsmittel verwendet werden.

Beispiele für solche Lösungsmittel oder Verdünnungsmittel sind Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isoproylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Dieses Verfahren gemäss der vorliegenden Erfindung kann bei einer Temperatur innerhalb eines weiten Bereichs durchgeführt werden, im allgemeinen bei einer Temperatur zwischen etwa −20° C und dem Siedepunkt der Mischung, vorzugsweise zwischen etwa 0° C und etwa 100° C. Zweckmässigerweise wird die Reaktion unter atmosphärischem Druck durchgeführt, jedoch ist es auch möglich, unter erhöhtem oder vermindertem Druck zu arbeiten.

*Verfahren ii)*

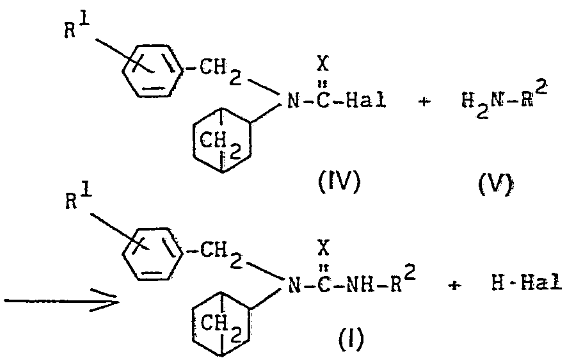

(IV) (V)

(I)

In den vorstehenden Formeln haben $R^1$, X, $R^2$ und Hal die im Vorstehenden angegebenen Bedeutungen.

In dem Reaktionsschema sind spezielle Beispiele für $R^1$, X und $R^2$ die gleichen, wie sie bereits im Vorstehenden genannt wurden. Spezielle Beispiele für Hal sind die gleichen Halogen-Atome, die im Vorstehenden erwähnt wurden, vorzugsweise Chlor und Brom.

In dem durch das vorstehende Reaktionsschema dargestellten Verfahren zählen zu speziellen Beispielen für das Carbamoylhalogenid der allgemeinen Formel (IV) als Ausgangsmaterial

N-4-Chlorobenzyl-N-2-norbornyl-carbamoylchlorid,

N-4-Bromobenzyl-N-2-norbornyl-carbamoylchlorid,

N-4-Methylbenzyl-N-2-norbornyl-carbamoylchlorid,

N-4-Clorobenzyl-N-2-norbornylthio-carbamoylchlorid,

N-4-Bromobenzyl-N-2-norbornylthio-carbamoylchlorid und

N-4-Methylbenzyl-N-2-norbornylthio-carbamoylchlorid.

Die entsprechenden Bromide kommen hierfür ebenfalls in Betracht.

Zu speziellen Beispielen für das Amin der allgemeinen Formel (V), das in gleicher Weise ein Ausgangsmaterial ist, zählen

Methylamin,
Ethylamin,
Propylamin,
Isopropylamin,
n-Butylamin,
n-Hexylamin,
Cyclopentylamin,
Cyclohexylamin,
Anilin,
2-Chloroanilin,
4-Chloroanilin,
4-Hydroxyanilin,
o-Toluidin,
p-Toluidin und
3-Methoxyanilin.

Verbindungen der allgemeinen Formel (I), in denen $R^2$ eine durch Hydroxyl substituierte Phenyl-Gruppe ist, werden vorzugsweise nach dem Verfahren i) hergestellt.

Das vorstehend genannte Verfahren ii) wird an einem typischen Beispiel im einzelnen beschrieben.

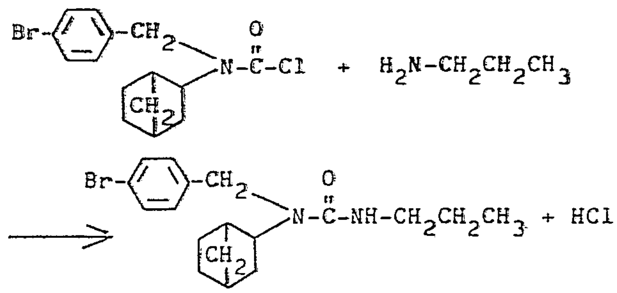

Das Verfahren ii) kann unter Verwendung der gleichen inerten Lösungsmittel oder Verdünnungsmittel durchgeführt werden, wie sie im Vorstehenden beispielhaft genannt wurden, wodurch das angestrebte Produkt in hoher Reinheit und Ausbeute erhalten wird.

Die im Vorstehenden beschriebene Reaktion kann in Gegenwart eines säurebindenden Mittels durchgeführt werden. Beispiele für solche säurebindenden Mittel sind die Hydroxide, Carbonate, Hydrogencarbonate und Alkoholate von Alkalimetallen sowie tertiäre Amine wie Triethylamin, Diethylanilin und Pyridin, die sämtlich im Normalfalle Verwendung finden.

Das im Vorstehenden beschriebene Verfahren kann bei einer Temperatur innerhalb eines weiten Bereichs durchgeführt werden, im allgemeinen bei einer Temperatur zwischen etwa −20° C und dem Siedepunkt der Mischung, vorzugsweise zwischen etwa 0° C und etwa 100° C. Zweckmässigerweise wird die Reaktion unter atmosphärischem Druck durchgeführt, jedoch ist es auch möglich, unter erhöhtem oder vermindertem Druck zu arbeiten.

Für den Einsatz als Fungizid in Landwirtschaft und Gartenbau können die Verbindungen gemäss der vorliegenden Erfindung als solche nach direkter Verdünnung mit Wasser oder nach ihrer Formulierung zu einem der verschiedenen Wirkstoffpräparate eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden die fungiziden Mittel in Form ihrer verschiedenen Präparate entweder unmittelbar oder nach dem Verdünnen mit Wasser auf die gewünschte Konzentration eingesetzt.

Beispiele für die landwirtschaftlich unbedenklichen Hilfsstoffe, auf die hier Bezug genommen wird, sind Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z.B n-Hexan, Petrolether, Naphtha, Erdöl-Fraktionen (z.B. Paraffinwachse, Kerosin, Leichtöle, Mittelöle, Schweröle), Benzol, Toluol und Xylole], halogenierte Kohlenwasserstoffe (z.B. Methylenchlorid, Kohlenstofftetrachlorid, Trichloroethylen, Ethylenchlorid, Ethylendibromid, Chlorbenzol und Chloroform), Alkohole (z.B. Methylalkohol, Ethylalkohol, Propylalkohol und Ethylenglycol), Ether (z.B. Ethylether, Ethylenoxid und Dioxan), Alkohol-ether (z.B. Ethylenglycolmonomethylether), Ester (z.B. Ethylacetat und Amylacetat), Ketone (z.B. Aceton und Isophoron), Amide (z.B. Dimethylformamid und Dimethylacetamid) und Sulfoxide (z.B. Dimethylsulfoxid).

Beispiele für Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anioinische oberflächenaktive Mittel wie Alkylschwefelsäureester (z.B. Natriumlaurylsulfat), Arylsulfonsäure-Salze (z.B. Alkylarylsulfonsäure-Salze und Natriumalkylnapthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfetsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas und niedere Ether), Verbrennungsregulatoren für Räuchermittel (z.B. Nitrite, Zink-Pulver und Dicyandiamid), Sauerstoff liefernde Mittel wie Salze der Chlorsäure und Salze der Dichromsäure), wirkungsverlängernde Mittel, Dispersions-Stabilisatoren (z.B. Casein, Tragant, Carboxymethylcellulose und Polyvinylalkohol) und synergistische Mittel.

Die Verbindungen der vorliegenden Erfindung können mittels der allgemein auf dem Gebiet der Agrochemikalien gebräuchlichen Verfahren in Form verschiedener Präparate formuliert werden. Beispiele für solche Anwendungsformen sind emulgierbare Konzentrate, Öle, benetzbare Pulver, lösliche Pulver, Suspensionen, Stäubemittel, Granulat, pulvrige Mittel, Räuchermittel, Tabletten, Aerosole, Pasten und Kapseln.

Die Fungizide für den Einsatz in Landwirtschaft und Gartenbau gemäss der vorliegenden Erfindung können von 0,1 bis 95 Gew.-%, vorzugsweise von 0,5 bis 90 Gew.-% des Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,0001 bis etwa 20 Gew.-%, vorzugsweise etwa 0,005 bis etwa 10 Gew.-%.

Der Gehalt des Wirkstoffs kann in geeigneter Weise je nach der Art des Präparats oder Mittels, Verfahren, Zweck, Zeit und Ort seiner Anwendung, dem Befallszustand der erkrankten Pflanzen etc. variiert werden.

Erforderlichenfalls können die Verbindungen gemäss der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen [Organophosphorester-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen] und/oder Düngemitteln.

Die den im Vorstehenden bezeichneten Wirkstoff enthaltenen verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung und Giessen), Räuchern, Bodenbehandlung (Vermischen, Streuen, Verdampfen und Injizieren), Oberflächenbehandlung (Beschichten, Aufbringen von Streifen, Aufstäuben und Bedecken) und Eintauchen. Sie können auch mittels des sogenannten Ultra-Low-Volume-Sprühverfahrens aufgebracht werden. Nach diesem Verfahren kann der Wirkstoff sogar in einer Konzentration von 100% zur Anwendung gelangen.

Die Aufwandmengen pro Flächeneinheit beträgt beispielsweise etwa 0,03 bis etwa 10 kg/ha, vorzugsweise etwa 0,3 bis etwa 6 kg/ha. In besonders gelagerten Fällen können oder müssen sogar die Aufwandmengen ausserhalb des angegebenen Bereichs liegen.

Gemäss der vorliegenden Erfindung kann ein fungizides Mittel für den Einsatz in der Landwirtschaft und im Gartenbau zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Bekämpfung von Pflanzenkrankheiten verfügbar, das darin besteht, dass eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf einen pathogenen Pilz und/oder den Ort, auf dem dieser Pilz oder eine durch diesen hervorgerufene Erkrankung auftritt, aufgebracht wird.

Die vorliegende Erfindung wird durch die folgenden Beispiele näher erläutert, ist jedoch nicht auf diese beschränkt.

*Beispiel 1*

*Benetzbares Pulver.*

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat wurden pulverisiert und zu einem benetzbaren Pulver vermischt. Dieses wurde mit Wasser verdünnt und auf einen pathogenen Pilz und/oder den Ort, auf dem der Pilz oder eine durch diesen verursachte Krankheit auftrat, aufgebracht.

*Beispiel 2*

*Emulgierbares Konzentrat.*

30 Teile der Verbindung Nr. 2 der Erfindung, 55 Teile Xylol, 8 Teile Polyoxyethylen-alkylphenylether und 7 Teile Calciumalkylbenzolsulfonat wurden unter Rühren miteinander vermischt, wodurch ein emulgierbares Konzentrat hergestellt wurde. Dieses wurde mit Wasser verdünnt und auf einen pathogenen Pilz und/oder den Ort, auf dem der Pilz oder eine durch diesen verursachte Krankheit auftrat, aufgebracht.

*Beispiel 3*

*Stäubemittel.*

2 Teile der Verbindung Nr. 3 der Erfindung und 98 Teile Tonpulver wurden pulverisiert und gemischt, wodurch ein Stäubemittel hergestellt wurde. Dieses wurde über einem pathogenen Pilz und/oder dem Ort, auf dem der Pilz oder eine durch diesen verursachte Krankheit auftrat, ausgestreut.

*Beispiel 4*

*Stäubemittel.*

Die Verbindung Nr. 4 der Erfindung (1,5 Teile), 0,5 Teil Isopropylhydrogenphosphat und 98 Teile Tonpulver wurden pulverisiert und gemischt, wodurch ein Stäubemittel hergestellt wurde. Dieses wurde über einem pathogenen Pilz und/oder dem Ort, auf dem der Pilz oder eine durch diesen verursachte Krankheit auftrat, ausgestreut.

*Beispiel 5*

*Granulat.*

Wasser (25 Teile) wurde zu einer Mischung aus 10 Teilen der Verbindung Nr. 5 der Erfindung, 30 Teilen Bentonit (Montmorillonit), 58 Teilen Talkum und 2 Teilen Ligninsulfonat zugesetzt, und das Gemisch wurde gut geknetet. Die Mischung wurde mittels eines Extruder-Granulators zu einem Granulat mit einer Korngrösse von 0,43 bis 2,0 mm (10 bis 40 mesh) verarbeitet und bei 40° C bis 50° C getrocknet, wodurch ein Granulat hergestellt wurde. Dieses wurde über einem pathogenen Pilz und/oder dem Ort, auf dem der Pilz oder eine durch diesen verursachte Krankheit auftrat, ausgestreut.

*Beispiel 6*

*Granulat.*

Ein Drehmischer wurde mit 95 Teilen Tonmineral-Teilchen mit einer Teilchengrössen-Verteilung im Bereich von 0,2 bis 2 mm beschickt, und unter Drehen des Mischers wurden 5 Gew.-Teile der Verbindung Nr. 6 der Erfindung, gelöst, in einem organischen Lösungsmittel gleichmässig auf die Tonmineral-Teilchen aufgesprüht. Die Tonmineral-Teilchen wurden dann bei 40° C bis 50° C getrocknet, wodurch ein Granulat gebildet wurde. Das Granulat wurde über einem pathogenen Pilz und/oder dem Ort, auf dem der Pilz oder eine durch diesen verursachte Krankheit auftrat, ausgestreut.

*Beispiel 7*

*Öl.*

Die Verbindung Nr. 7 der Erfindung (0,5 Teil) und 99,5 Teile Kerosin werden unter Rühren vermischt, wodurch ein Öl gebildet wird. Es wird auf einen pathogenen Pilz und/oder dem Ort, auf dem der Pilz oder eine durch diesen verursachte Krankheit auftrat, aufgesprüht.

Die ausgezeichnete fungizide Wirkung der aktiven Verbindungen gemäss der vorliegenden Erfindung ist aus den Ergebnissen der folgenden Test-Beispiele zu entnehmen.

*Test-Beispiel 1*

Test der Bekämpfungswirkung gegen Schalenmehltau (Pellicularia sasakii; «sheath blight») (Topf-Test):

Herstellung des Test-Präparates:

Wirkstoff: 50 Gew.-Teile;

Träger: 45 Gew.-Teile einer Mischung (1:5) aus Diatomeenerde und Kaolin;

Emulgator: 5 Gew.-Teile Polyoxyethylenalkylphenylether.

Der Wirkstoff, der Träger und der Emulgator wurden in den angegebenen Mengen miteinander vermischt, wodurch ein benetzbares Pulver gebildet wurde. Eine vorher festgelegte Menge des benetzbaren Pulvers wurde mit Wasser verdünnt, wodurch ein Test-Präparat gebildet wurde.

Test-Verfahren:

Reis (Varietät: Kinmaze) wurde in Wagner-Töpfen (2 $dm^2$) im bewässerten Zustand kultiviert. Im frühen Stadium der Ähren-Bildung wurde eine wie im Vorstehenden angegeben hergestellte Verdünnung des Test-Präparates in vorherbestimmter Konzentration in einer Menge von 100 ml auf jeweils 3 Töpfe aufgesprüht. Am Tage nach dem Sprühen wurde der Pflanzhügel am unteren Ende der Reispflanze mit einem den Schalenmehltau verursachenden Pilz (Pellicularia sasakii) beimpft, der 10 Tage in einem Gerste-Medium bis zur Bildung von Sklerotia kultiviert worden war. Die Reispflanze wurde 10 Tage in einer Kammer gehalten, in der eine Temperatur von 28° C bis 30° C und eine relative Luftfeuchtigkeit von wenigstens 95% aufrechterhalten wurden, um den Schalenmehltau auszulösen. Dann wurden der Grad der Erkrankung und das Auftreten von Phytotoxizität untersucht. Der Grad der Schädigung wurde mit Hilfe des nachstehenden Standards angegeben, der ermittelt wurde nach dem Grad der Ausdehnung der Schädigung von den beimpften Teilen der Wurzeln her.

$$\text{Grad der Schädigung} = \frac{3n_3+2n_2+n_1+n_0}{3\,N} \times 100$$

Hierin bedeuten

N die Gesamtzahl der untersuchten Stengel,

$n_0$ die Zahl der durch die Erkrankung nicht infizierten Stengel,

$n_1$ die Zahl der Stengel, die (vom Boden her) bis hinauf zu einer Schale des ersten Blattes befallen waren,

$n_2$ die Zahl der Stengel, die bis hinauf zu einer Schale des zweiten Blattes befallen waren, und

$n_3$ die Zahl der Stengel, die bis hinauf zu einer Schale des dritten Blattes befallen waren.

Die Ergebnisse sind in der Tabelle 1 dargestellt.

*Tabelle 1*

| Verbindung[1] Nr. | Wirkstoff-Konzentration (ppm) | Grad der Schädigung | Phytotoxizität[4] |
|---|---|---|---|
| 1 | 25 | 0 | — |
| 2 | 125 | 0 | — |
| 3 | 25 | 0 | — |
| 4 | 25 | 0 | — |
| 5 | 25 | 0 | — |
| 7 | 25 | 0 | — |
| 8 | 25 | 0 | — |
| 11 | 125 | 0 | — |
| 13 | 125 | 0 | — |
| 14 | 125 | 0 | — |
| 15 | 250 | 0 | — |
| 17 | 25 | 0 | — |
| 18 | 125 | 0 | — |
| 19 | 25 | 0 | — |
| 20 | 25 | 0 | — |
| 21 | 25 | 0 | — |
| 22 | 250 | 0 | — |
| 23 | 25 | 0 | — |

| Verbindung[1) ] Nr. | Wirkstoff-Konzentration (ppm) | Grad der Schädigung | Phytotoxizität[4)] |
|---|---|---|---|
| 24 | 25 | 0 | — |
| 25 | 25 | 0 | — |
| 26 | 25 | 0 | — |
| 27 | 25 | 0 | — |
| 28 | 250 | 0 | — |
| Polyoxin[2)] | 45 | 21,5 | — |
| Validamycin A[3)] | 60 | 10 | — |

[1)] Die Verbindungs-Nr. in der Tabelle entspricht derjenigen in den betreffenden Synthese-Beispielen und in der Tabelle 4.
[2)] Polyoxin: Zink-Salz der Polyoxin-Verbindung.
[3)] Validamycin A (Handelsprodukt, das für den Kontroll-Versuch verwendet wurde): 3-proz. flüssiges Präparat von Validamycin A.
[4)] Das Symbol — in der Spalte «Phytotoxizität» der Tabelle gibt an, dass keine Phytotoxizität auftrat.

*Test-Beispiel 2*

Test der Wirkung der Wasser-Oberflächen-Anwendung auf die Bekämpfung des Reis-Schalenmehltaus:

Test-Verfahren:

Drei Wurzelstöcke von Reis (Varietät: Nihonbare) wurden jeweils in einen weissen Porzellan-Topf von 12 cm Durchmeser gepflanzt und im bewässerten Zustand kultiviert. Im Anfangsstadium des Auftretens neuer Triebe wurde eine Zubereitung des Test-Präparats, die mit vorher festgelegter Konzentration in gleicher Weise wie in Beispiel 1 hergestellt worden war, in den angegebenen Mengen mittels einer Pipette auf die Wasseroberfläche gegeben, so dass das Test-Präparat nicht auf die an der Bodenoberfläche befindlichen Teile der Reispflanzen spritzte. Fünf Tage später wurde das in dem Topf enthaltene Wasser entfernt, und die Oberfläche des Bodens wurde mit Perlit bedeckt. Ein Schalenmehltau-Pilz, der nach 10-tägiger Kultivierung in einem Gerste-Medium Sklerotia enthielt, wurde am unteren Ende der zu untersuchenden Reispflanzen aufgeimpft. Danach wurde der Topf 24 h in einem Inkubator gehalten, in dem eine Temperatur von 23° C bis 25° C und eine relative Luftfeuchtigkeit von 100% aufrechterhalten wurden. Danach wurde der Topf in ein Gewächshaus aus Glas mit einer Temperatur von 20° C bis 28° C überführt. Am siebten Tage nach der Beimpfung wurde die gleiche Prüfung wie im Test-Beispiel 1 durchgeführt, und der Grad der Schädigung und der Phytotoxizität wurden bestimmt.

Die Ergebnisse sind in der Tabelle 2 dargestellt.

*Tabelle 2*

| Verbindung[1)] Nr. | Wirkstoff-Konzentration ($g/m^2$) | Grad der Schädigung | Phytotoxizität[2)] |
|---|---|---|---|
| 3 | 0,5 | 0 | — |
| 5 | 0,5 | 0 | — |
| 15 | 0,5 | 1,0 | — |
| 19 | 0,5 | 0,5 | — |

[1)] In Tabelle 2 bezeichnet die Verbindungs-Nr. die entsprechend numerierte Verbindung der Tabelle 1.
[2)] Das Symbol — in der Spalte «Phytotoxizität» der Tabelle gibt an, dass keine Phytotoxizität auftrat.

*Test-Beispiel 3*

Test der Bekämpfungswirkung gegen Triebfäule (im Gewächshaus):

Hierbei handelt es sich um ein Beispiel für die Bekämpfungswirkung aufgrund einer Bodenbehandlung gegen Rhizoctonia solani, einen sich im Boden ausbreitenden pathogenen Pilz, der die Triebfäule verschiedenartiger Nutzpflanzen hervorruft.

Herstellung des Test-Präparates:

3 Gew.-Teile des Wirkstoffs und 97 Gew.-Teile Talkum wurden pulverisiert und miteinander vermischt.

Test-Verfahren:

Ein durch Autoklavieren sterilisierter landwirtschaftlicher Ackerboden wurde mit Rhizoctonia solani beimpft, das 10 Tage in einem Kleie-Medium kultiviert worden war, wodurch ein entsprechend verseuchter Boden präpariert wurde. Das wie im Vorstehenden angegeben hergestellte Stäubemittel wurde in vorher festgelegter Konzentration mit dem verseuchten Boden vermischt, und die Mischung wurde zur Durchführung der Bodenbehandlung gut durchgerührt. Der so behandelte Boden sowie unbehandelter Boden zu Vergleichszwecken wurden jeweils in Kunststoffbehälter mit einer Fläche von 27 × 18 $cm^2$ und einer Tiefe von 9 cm eingefüllt, so dass Saatbetten erhalten wurden. 50 Samenkörner von Gurken und 50 Samenkörner von Auberginen wurden jeweils in einen Behälter eingesät und in einem Gewächshaus den üblichen Anzuchtbedingungen ausgesetzt, um die Keimung herbeizuführen. Die Anzahl Sämlinge, die an vorher festgelegten Tagen von der Triebfäule befallen waren, sowie das Auftreten von Phytotoxizität gegenüber dem Wachstum der Gemüsepflanzen wurden untersucht. Am 25. Tage nach der Einsaat wurden die Ergebnisse insgesamt ausgewertet.

Die Ergebnisse sind in der Tabelle 3 dargestellt.

*Tabelle 3*

| Verbindung[1] Nr. | Wirkstoff-Konzentration[2] (ppm) | Gurken | | Auberginen | |
|---|---|---|---|---|---|
| | | Triebfäule-Anteil (%) | Phytotoxizität[4] | Triebfäule-Anteil (%) | Phytotoxizität[4] |
| 15 | 12,5 | 2,0 | — | 0 | — |
| | 25 | 0 | — | 0 | — |
| | 50 | 0 | — | 0 | — |
| 18 | 25 | 5,0 | — | 2,0 | — |
| | 50 | 0 | — | 0 | — |
| 19 | 25 | 7,5 | — | 5,0 | — |
| | 50 | 0 | — | 0 | — |
| PCNB[3] | 50 | 10,0 | — | 5,0 | — |
| unbehandelt | — | 100 | | 100 | |

[1] In Tabelle 3 bezeichnet die Verbindungs-Nr. die entsprechend numerierte Verbindung der Tabelle 1.
[2] Die Konzentration des Wirkstoffs gibt das Verhältnis des Gewichts des Wirkstoffs zu dem Volumen des Bodens an.
[3] PCNB = Pentachloronitrobenzol.
[4] Das Symbol — in der Spalte «Phytotoxizität» der Tabelle gibt an, dass keine Phytotoxizität auftrat.

Die Verfahren zur Herstellung der Verbindungen gemäss der vorliegenden Erfindung werden im einzelnen mit Hilfe der nachstehenden Synthese-Beispiele beschrieben.

*Synthese-Beispiel 1*

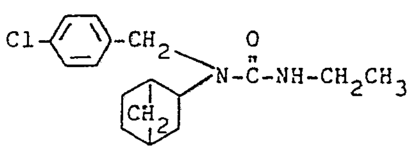

(Verbindung Nr. 1)

24 g N-4-Chlorobenzyl-2-norbornylamin wurden in 400 ml Hexan gelöst, und unter Rühren und Kühlung wurde eine Lösung von 8 g Ethylisocyanat in 30 ml Hexan tropfenweise zu der Lösung hinzugefügt. Nach der Zugabe wurde die Temperatur der Mischung allmählich erhöht, und sie wurde etwa 3 h bei 40° C gerührt. Nach dem Abkühlen wurden die ausgefallenen Kristalle unter Absaugen filtriert und aus einer Mischung von Hexan und Ethanol umkristallisiert, wonach 26,7 g des angestrebten 1-(4-Chlorobenzyl)-3-ethyl-1-(2-norbornyl)harnstoffs erhalten wurden; Schmp. 132-133° C.

*Synthese-Beispiel 2*

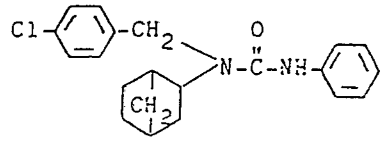

(Verbindung Nr. 19)

Anilin (19 g) wurde in 400 ml Toluol gelöst, und unter Rühren und Kühlung wurde eine Lösung von 30 g N-4-Chlorobenzyl-N-2-norbornylcarbamoylchlorid in 50 ml Toluol tropfenweise hinzugefügt. Nach der Zugabe wurde die Temperatur der Mischung allmählich erhöht, und sie wurde etwa 10 h bei 70° C bis 80° C gerührt. Nach dem Abkühlen wurde das ausgefallene Anilinhydrochlorid durch Filtration abgetrennt. Die Toluol-Schicht wurde nacheinander mit Wasser, einer 1-proz. wässerigen Lösung von Natriumcarbonat, 1-proz. Salzsäure und wiederum mit Wasser gewaschen und danach über wasserfreiem Natriumsulfat getrocknet. Das Toluol wurde abgedampft, und

der Rückstand wurde aus einer Mischung von Hexan und Ethanol umkristallisiert, wonach 23 g des angestrebten 1-(4-Chlorobenzyl)-1-(2-norbornyl)-3-phenylharnstoffs erhalten wurden; Schmp. 111-113° C.

Verbindungen gemäss der vorliegenden Erfindung, die nach im wesentlichen den gleichen Verfahren wie in den Synthese-Beispielen 1 und 2 synthetisiert wurden, sind in der Tabelle 4 aufgeführt.

*Tabelle 4*

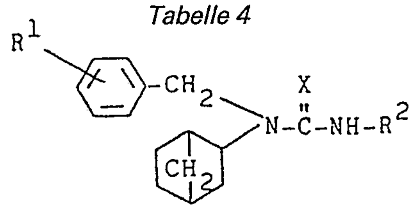

| Verbindung Nr. | $R^1$ | X | $R^2$ | Physikalische Konstante (Schmp., °C) |
|---|---|---|---|---|
| 2 | 4-Cl | O | $-CH_3$ | 121-123 |
| 3 | 4-Cl | O | n-$C_3H_7$ | 95- 96 |
| 4 | 4-Cl | O | iso-$C_3H_7$ | 125-126 |
| 5 | 4-Cl | O | n-$C_4H_9$ | 88- 89 |
| 6 | 4-Cl | O | n-$C_6H_{13}$ | 86- 87 |
| 7 | 4-Cl | O | 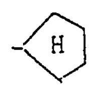 | 98- 99 |
| 8 | 4-Cl | O | 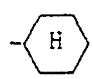 | 115-117 |
| 9 | 4-Cl | O | 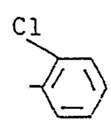 | 111-113 |
| 10 | 4-Cl | O | 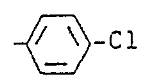 | 151-153 |
| 11 | 4-Cl | O | 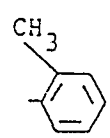 | 133-134 |
| 12 | 4-Cl | O | 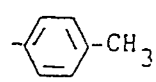 | 127-129 |
| 13 | 4-Cl | O | 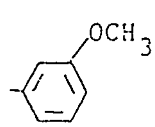 | 126-127 |

*Tabelle 4* (Fortsetzung)

| Verbindung Nr. | $R^1$ | X | $R^2$ | Physikalische Konstante (Schmp., °C) |
|---|---|---|---|---|
| 14 | 4-Br | O | $-CH_3$ | 136-137 |
| 15 | 4-Br | O | $n\text{-}C_3H_7$ | 80- 81 |
| 16 | 4-Br | O | $n\text{-}C_6H_{13}$ | 76- 78 |
| 17 | 4-Cl | S | $-CH_3$ | 124-125 |
| 18 | 4-Cl | S | $-C_2H_5$ | 70- 73 |
| 20 | 4-Br | O | 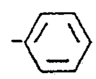 | 132-133 |
| 21 | $4\text{-}CH_3$ | O | $-C_2H_5$ | 98- 99 |
| 22 | $4\text{-}CH_3$ | O | $n\text{-}C_3H_7$ | 74- 75 |
| 23 | $4\text{-}CH_3$ | O | $n\text{-}C_4H_9$ | 49- 52 |
| 24 | $4\text{-}CH_3$ | O | 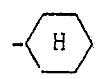 | 89- 90 |
| 25 | $4\text{-}CH_3$ | O | 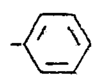 | 121-122 |
| 26 | 4-Cl | S | $n\text{-}C_4H_9$ | 99-101 |
| 27 | 4-Br | S | $-CH_3$ | 119-120 |
| 28 | 4-Cl | O | 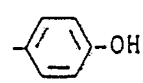 | 178-180 |

**Patentansprüche**

1. Benzylharnstoff-Derivate der allgemeinen Formel

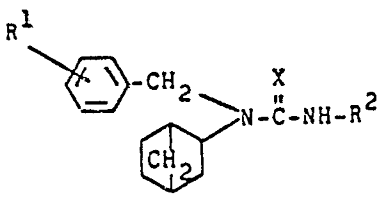

in der

$R^1$ ein Halogen-Atom oder eine Alkyl-Gruppe mit 1 bis 4 Kohlenstoffatomen bezeichnet,

X ein Sauerstoff- oder Schwefel-Atom bezeichnet und

$R^2$ eine Alkyl-Gruppe mit 1 bis 8 Kohlenstoffatomen, eine Cycloalkyl-Gruppe mit 5 bis 8 Kohlenstoffatomen oder eine Phenyl-Gruppe, die durch mindestens einen Substituenten aus der aus Halogen-Atomen, Alkyl-Gruppen mit 1 bis 4 Kohlenstoffatomen, Alkoxy-Gruppen mit 1 bis 4 Kohlenstoffatomen und Hydroxyl-Gruppen bestehenden Klasse substituiert sein kann, bezeichnet.

2. Benzylharnstoff-Derivate nach Anspruch 1, wobei

$R^1$ für Fluor, Chlor, Brom, Jod oder für Alkyl mit 1 bis 4 Kohlenstoffatomen steht,

X für ein Sauerstoff- oder Schwefelatom steht und

$R^2$ für Alkyl mit 1 bis 8 Kohlenstoffatomen, für Cycloalkyl mit 5 bis 8 Kohlenstoffatomen oder für gegebenenfalls ein- bis fünffach durch Fluor, Chlor, Brom, Jod, Alkyl mit 1 bis 4 Kohlenstoffatomen, Alkoxy mit 1 bis 4 Kohlenstoffatomen oder Hydroxyl substituiertes Phenyl steht.

3. Benzylharnstoff-Derivate nach Anspruch 1, wobei

$R^1$ für Chlor, Brom, Methyl oder Ethyl steht,

X für ein Sauerstoff- oder Schwefelatom steht und

$R^2$ für Alkyl mit 1 bis 8 Kohlenstoffatomen, für Cycloalkyl mit 5 bis 8 Kohlenstoffatomen oder für gegebenenfalls durch Fluor, Chlor, Brom, Jod, Alkyl mit 1 bis 4 Kohlenstoffatomen, Alkoxy mit 1 bis 4 Kohlenstoffatomen oder Hydroxyl substituiertes Phenyl steht.

4. Verfahren zur Herstellung von Benzylharnstoff-Derivaten der allgemeinen Formel

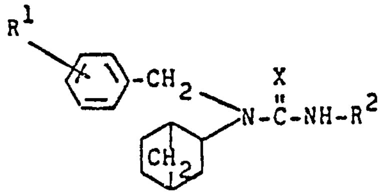

(I)

in der

$R^1$ ein Halogen-Atom oder eine Alkyl-Gruppe mit 1 bis 4 Kohlenstoffatomen bezeichnet,

X ein Sauerstoff- oder Schwefel-Atom bezeichnet und

$R^2$ eine Alkyl-Gruppe mit 1 bis 8 Kohlenstoffatomen, eine Cycloalkyl-Gruppe mit 5 bis 8 Kohlenstoffatomen oder eine Phenyl-Gruppe, die gegebenenfalls durch Halogen, Alkyl-Gruppen mit 1 bis 4 Kohlenstoffatomen, Alkoxy-Gruppen mit 1 bis 4 Kohlenstoffatomen und Hydroxyl-Gruppen substituiert sein kann,

dadurch gekennzeichnet, dass man

a) ein Amin der allgemeinen Formel

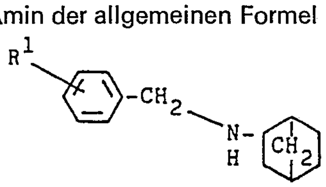

(II)

in der $R^1$ die im Vorstehenden angegebene Bedeutung hat, mit einem Isocyanat der allgemeinen Formel

$$R^2-N=C=X \qquad (III)$$

in der X und $R^2$ die im Vorstehenden angegebenen Bedeutungen haben, gegebenenfalls in Gegenwart eines Lösungsmittels umsetzt oder

b) dass man ein Carbamoylhalogenid der allgemeinen Formel

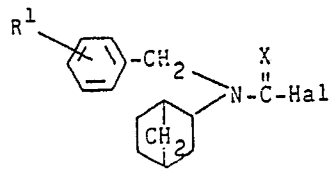

(IV)

in der $R^1$ und X die im Vorstehenden angegebenen Bedeutungen haben, Hal für Halogen steht, mit einem Amin der allgemeinen Formel

$$H_2N-R^2 \qquad (V)$$

in der $R^2$ die im Vorstehenden angegebene Bedeutung hat, gegebenenfalls in Gegenwart eines Lösungsmittels und gegebenenfalls in Gegenwart eines Säureakzeptors umsetzt.

5. Fungizide Mittel, gekennzeichnet durch einen Gehalt an mindestens einem Benzylharnstoff-Derivat der Formel (I) gemäss Ansprüchen 1 und 4.

6. Verwendung von Benzylharnstoff-Derivaten der Formel (I) gemäss Ansprüchen 1 und 4 zur Bekämpfung von Pilzen.

7. Bakterizide Mittel, gekennzeichnet durch einen Gehalt an mindestens einem Benzylharnstoff-Derivat der Formel (I) gemäss Ansprüchen 1 und 4.

8. Verwendung von Benzylharnstoff-Derivaten der Formel (I) gemäss Ansprüchen 1 und 4 zur Bekämpfung von Bakterien.

## Claims

1. Benzylurea derivatives of the general formula

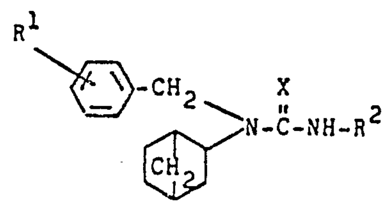

in which

$R^1$ designates a halogen atom or an alkyl group with 1 to 4 carbon atoms,

X designates an oxygen or sulphur atom and

$R^2$ designates an alkyl group with 1 to 8 carbon atoms, a cycloalkyl group with 5 to 8 carbon atoms or a phenyl group which can be substituted by at least one substituent from the class consisting of halogen atoms, alkyl groups with 1 to 4 carbon atoms, alkoxy groups with 1 to 4 carbon atoms and hydroxyl groups.

2. Benzylurea derivatives according to Claim 1, wherein

$R^1$ represents fluorine, chlorine, bromine, iodine or alkyl with 1 to 4 carbon atoms,

X represents an oxygen or sulphur atom and

$R^2$ represents alkyl with 1 to 8 carbon atoms, cycloalkyl with 5 to 8 carbon atoms or phenyl which is optionally mono- to pentasubstituted by fluorine, chlorine, bromine, iodine, alkyl with 1 to 4 carbon atoms, alkoxy with 1 to 4 carbon atoms or hydroxyl.

3. Benzylurea derivatives according to Claim 1, wherein

$R^1$ represents chlorine, bromine, methyl or ethyl,
X represents an oxygen or sulphur atom and
$R^2$ represents alkyl with 1 to 8 carbon atoms, cycloalkyl with 5 to 8 carbon atoms or phenyl which is optionally substituted by fluorine, chlorine, bromine, iodine, alkyl with 1 to 4 carbon atoms, alkoxy with 1 to 4 carbon atoms or hydroxyl.

4. Process for the preparation of benzylurea derivatives of the general formula

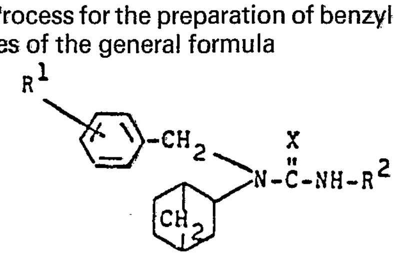
(I)

in which
$R^1$ designates a halogen atom or an alkyl group with 1 to 4 carbon atoms,
X designates an oxygen or sulphur atom and
$R^2$ an alkyl group with 1 to 8 carbon atoms, a cycloalkyl group with 5 to 8 carbon atoms or a phenyl group which can optionally be substituted by halogen, alkyl groups with 1 to 4 carbon atoms, alkoxy groups with 1 to 4 carbon atoms and hydroxyl groups,
characterized in that
a) an amine of the general formula

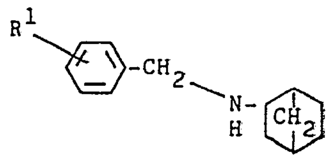
(II)

in which
$R^1$ has the aforementioned meaning, is reacted with an isocyanate of the general formula

$$R^2-N=C=X \qquad (III)$$

in which
X and $R^2$ have the aforementioned meanings, if appropriate in the presence of a solvent or
b) in that a carbamoyl halide of the general formula

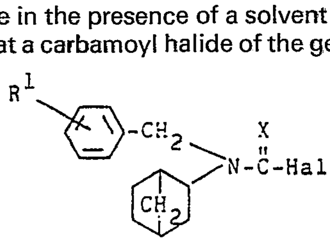
(IV)

in which
$R^1$ and X have the aforementioned meanings, and Hal represents halogen,
is reacted with an amine of the general formula

$$H_2N-R^2 \qquad (V)$$

in which
$R^2$ has the aforementioned meaning, if appropriate in the presence of a solvent and if appropriate in the presence of an acid acceptor.

5. Fungicidal agents, characterized in that they contain at least one benzylurea derivative of the formula (I) according to Claims 1 and 4.

6. Use of benzylurea derivatives of the formula (I) according to Claims 1 and 4 for combating fungi.

7. Bactericidal agents, characterized in that they contain at least one benzylurea derivative of the formula (I) according to Claims 1 and 4.

8. Use of benzylurea derivatives of the formula (I) according to Claims 1 and 4 for combating bacteria.

## Revendications

1. Dérivés de benzylurée de formule générale:

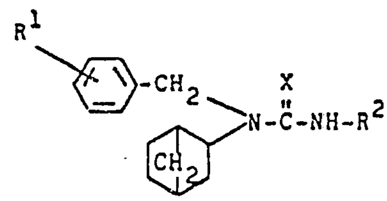

dans laquelle
$R^1$ représente un atome d'halogène ou un groupe alkyle contenant 1 à 4 atomes de carbone,
X représente un atome d'oxygène ou un atome de soufre, et
$R^2$ représente un groupe alklye contenant 1 à 18 atomes de carbone, un groupe cycloalkyle contenant 5 à 8 atomes de carbone ou un groupe phényle qui peut être substitué par au moins un substituant choisi parmi la classe comprenant les atomes d'halogènes, les groupes alkyle contenant 1 à 4 atomes de carbone, les groupes alcoxy contenant 1 à 4 atomes de carbone et le groupe hydroxy.

2. Dérivés de benzylurée selon la revendication 1, dans lesquels
$R^1$ représente un atome de fluor, un atome de chlore, un atome de brome, un atome d'iode ou un groupe alkyle conteannt 1 à 4 atomes de carbone,
X représente un atome d'oxygène ou un atome de soufre, et
$R^2$ représente un groupe alkyle contenant 1 à 8 atomes de carbone, un groupe cycloalkyle contenant 5 à 8 atomes de carbone ou un groupe phényle éventuellement substitué une à cinq fois par un atome de fluor, par un atome de chlore, par un atome de brome, par un atome d'iode, par un groupe alkyle contenant 1 à 4 atomes de carbone, par un groupe alcoxy contenant 1 à 4 atomes de carbone ou par un groupe hydroxy.

3. Dérivés de benzylurée selon la revendication 1, dans lesquels
$R^1$ représente un atome de chlore, un atome de brome, un groupe méthyle ou un groupe éthyle,
X représente un atome d'oxygène ou un atome de soufre, et
$R^2$ représente un groupe alkyle contenant 1 à 8 atomes de carbone, un groupe cyloalkyle contenant 5 à 8 atomes de carbone ou un groupe phényle éventuellement substitué par un atome de fluor, par un atome de chlore, par un atome de brome, par un atome d'iode, par un groupe alkyle contenant 1 à 4 atomes de carbone ou par un groupe hydroxy.

4. Procédé de préparation de dérivés de benzylurée de formule générale:

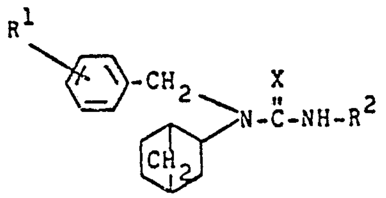

(I)

dans laquelle

$R^1$ représente un atome d'halogène ou un groupe alkyle contenant 1 à 4 atomes de carbone,

X représente un atome d'oxygène ou un atome de soufre, et

$R^2$ représente un groupe alkyle contenant 1 à 8 atomes de carbone, un groupe cycloalkyle contenant 5 à 8 atomes de carbone ou un groupe phényle qui peut éventuellement être substitué par un atome d'halogène, par des groupes alkyle contenant 1 à 4 atomes de carbone, par des groupes alcoxy contenant 1 à 4 atomes de carbone et par un groupe hydroxy, caractérisé en ce que:

a) on fait réagir une amine de formule générale:

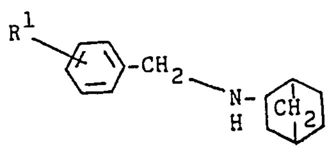

(II)

dans laquelle $R^1$ a la signification indiquée ci-dessus, avec un isocyanate de formule générale:

$$R^2-N=C=X \qquad (III)$$

dans laquelle X et $R^2$ ont les significations indiquées ci-dessus, éventuellement en présence d'un solvant, ou

b) on fait réagir un halogénure de carbamoyle de formule générale:

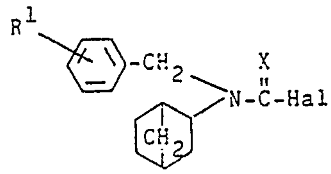

(IV)

dans laquelle $R^1$ et X ont les significations indiquées ci-dessus et Hal représente un atome d'halogène, avec une amine de formule générale:

$$H_2N-R^2 \qquad (V)$$

dans laquelle $R^2$ a la signification indiquée ci-dessus, éventuellement en présence d'un solvant et éventuellement en présence d'un accepteur d'acide.

5. Agents fongicides, caractérisés en ce qu'ils contiennent au moins un dérivé de benzylurée de formule (I) selon les revendications 1 et 4.

6. Utilisation de dérivés de benzylurée de formule (I) selon les revendications 1 et 4 en vue de combattre les champignons.

7. Agents bactéricides, caractérisés en ce qu'ils contiennent au moins un dérivé de benzylurée de formule (I) selon les revendications 1 et 4.

8. Utilisation de dérivés de benzylurée de formule (I) selon les revendications 1 et 4 en vue de combattre les bactéries.